# EUROPEAN PATENT APPLICATION

(11) **EP 0 911 014 A2**
(43) Date of publication of application: **28.04.1999**
(21) Application number: 98830605.6
(22) Date of filing: 13.10.1998
(51) Int. Cl.: A61G 15/14, A61B 19/02

(54) **A surgery furniture unit**

(30) Priority: 15.10.1997 IT BO970619
(71) Applicant: CASTELLINI S.p.A., I-40013 Castelmaggiore (Bologna) (IT)
(72) Inventor: Castellini, Franco, 40124 Bologna (IT)
(74) Representative: Lanzoni, Luciano

(57) **Abstract**

A furniture unit for dental surgeries comprises: a body (1) having, joined to each other, a first horizontal wall or bottom (2), a second horizontal wall (3) and third vertical sidewalls (4, 5, 6) fitted in relation to each other in such a way as to define an open, vertical zone (7) that gives access to the compartment thus formed; an element (8) for closing the zone (7) and consisting of a flexible panel connected on two sides to a pair of guides (9) that are joined to two opposite third sidewalls (4, 5). The panel (8) runs along the pair of guides (9) between a closed position where the panel (8) extends along, and covers, the zone (7) from end to end and an open position where the panel (8) has slid towards one end of the zone (7) and leaves the latter uncovered. To accomplish this, drive means (10) act on the panel (8) and allow it to assume two or more stable positions between the closed and open positions of the fourth wall (7).

## Description

The present invention relates to a furniture unit for a surgery, especially for a dental surgery, designed to hold medical instruments and consumables used by the dentist during treatment sessions.

At present, the wide range of hand instruments and consumable material that must be available to a dentist during a treatment session makes it necessary for the dental surgery to have one or more items of furniture, either fixed or equipped with means for moving them on the surgery floor.

These items of furniture are designed to simply contain the consumables or as a means which the instruments required during the session can be picked up from and replaced on.

Items of furniture used for this purpose have an outer body, typically parallelepipedal in shape, which may contain a series of shelves for the consumables, closed by one or more outward-opening doors, or a set of sliding drawer units especially fitted out to allow the dental surgeon or his/her assistant to conveniently pick up and replace the instruments (for example, mouth mirrors) used frequently during a treatment session with a patient.

Furniture units with this type of structure are based almost entirely on the design of standard office furniture and are not suitable to effectively and significantly reduce the propagation of germs within the surgery: that is because in a conventional furniture unit, the doors must be equipped with handles and knobs and the sliding drawers with special handgrips to enable the material to be accessed and picked up and then replaced. These items are therefore in continual contact with dentist's or the assistant's hands, creating areas (handles and knobs) where germs and bacteria can collect and thrive unless continually treated with sterilizers.

In addition to this, the hinges and runners of the mobile closing parts (doors and drawers) are subject to wear and, in time, are unable to guarantee sealed closure, thus allowing the passage of external agents even during periods when the instruments are not being used.

The Applicant, therefore, pursuing its consistent research policy of guaranteeing maximum hygiene in dental surgeries, has devised and produced a modular furniture unit especially for dental surgeries, designed to meet the diverse requirements of surgeries and to allow the material contained to be easily picked up and replaced under conditions of maximum hygiene with efficient and lasting means of opening and tight closure.

The technical characteristics of the invention according to the above mentioned aims are described in the claims below and its advantages are apparent from the detailed description which follows, with reference to the accompanying drawings which illustrate preferred embodiments of the invention and in which:
- Figure 1 is a front view of a furniture unit for dental surgeries made in accordance with the present invention;
- Figure 2 is a schematic side view, with some parts cut away in order to better illustrate others, of the furniture unit shown in Figure 1;
- Figure 3 illustrates in a schematic front view a composite item of furniture made from three modular units, each consisting of a furniture unit made in accordance with the present invention;
- Figure 4 is schematic front view of an enlarged detail of a part of Figure 2.
- Figure 5 is a side view, with some parts cut away in order to better illustrate others, of another embodiment of the composite item of furniture made from the units illustrated in the previous figures;
- Figure 6 is a front view of the composite item of furniture shown in Figure 5;
- Figure 7 is a perspective view of a furniture unit equipped with a fold-away wash-basin;
- Figure 8 is a perspective view of a furniture unit equipped with fold-away waste bin;
- Figure 9 is a scaled-up side view of a detail showing the base of a furniture made in accordance with the present invention equipped with an element for covering and housing control means.

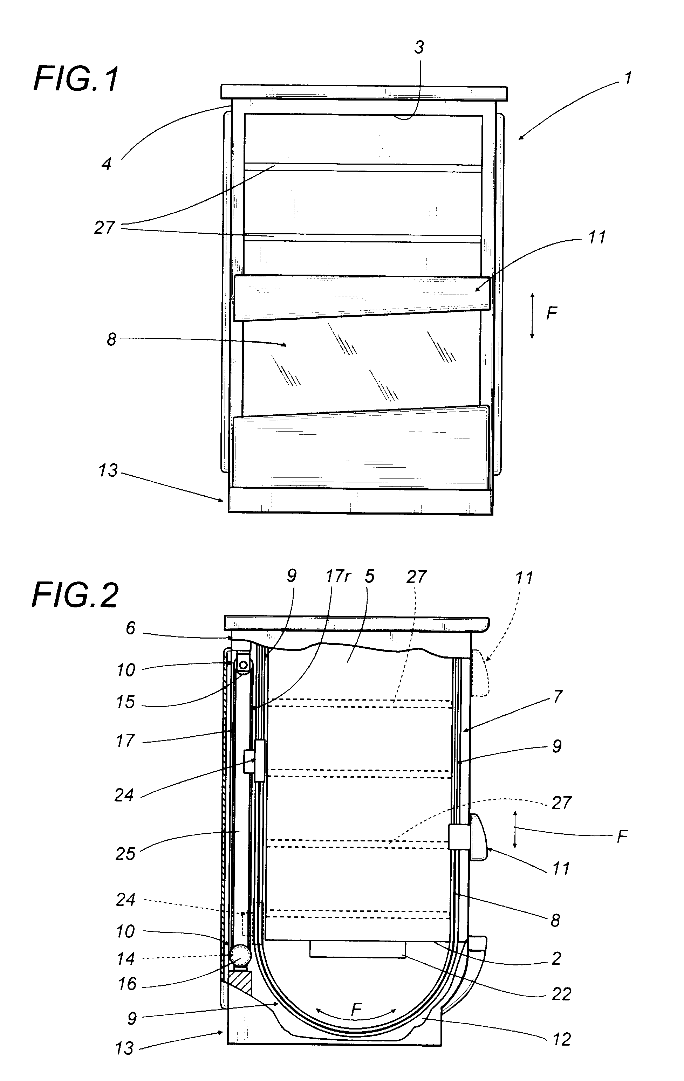

With reference to the accompanying drawings, and in particular Figures 1 and 2, the surgery furniture unit disclosed is designed especially, but not only, for dental surgeries.

The furniture unit, in its most simple form, comprises basically a body 1, an element 8 for closing a zone 7 of the body 1 and means 10 for driving the closing element 8.

In a first embodiment, the body 1, which defines the outer shell of the furniture unit, has, joined to each other, a first horizontal wall or bottom 2, a second horizontal wall or top 3 and third vertical sidewalls 4, 5, 6 fitted in relation to each other in such a way as to define a parallelepipedal body with an open, vertical zone 7 that gives access to the compartment thus defined inside the body 1.

The compartment defined inside the body 1 may be completely empty to simply provide space for the storage of consumables or it may be equipped with horizontal shelves 27, either fixed or movable on guides (according to the instruments to be housed) which are illustrated only schematically since they do not strictly form part of the invention. The compartment may also be used to house accessory elements (preferably fold-away) which will be described in more detail below.

The element 8 for closing the zone 7, which hereinafter will be referred to as fourth wall 7, consists of a flexible panel attached on two sides to a pair of guides 9 joined to and extending along two opposite third sidewalls 4 and 5.

The flexible panel 8 runs along the pair of guides 9 between two limit positions, of which one is a closed position where the panel 8 extends all the way along the fourth wall 7 and the other is an open position where the panel 8 has slid towards one end of the fourth wall 7.

Looking in more detail at the preferred embodiment, the flexible panel 8 consists of a single piece of sheet metal that runs along the pair of guides 9 on two sides: hence, the panel 8 is almost entirely inside the body 1 when it is in the position in which it leaves the fourth wall 7 open, following a path defined by the pair of guides 9 and partly lining the compartment inside the body 1.

As can be seen better in Figure 2, during the opening or closing of the fourth wall 7, the straight portion of panel 8 is deformed into a U shape as it follows the path formed by the guides 9 within the body 1, while the curved portion of the guides 9 is located in a gap 12 between the first horizontal wall 2 and a base 13 that supports the body 1: therefore, when it is driven, the panel 8 runs between the fourth wall 7 and the third vertical wall 6 opposite it, passing underneath the first wall 2.

Still with reference to Figures 1 and 2, the flexible panel 8 has a horizontal protuberance 11 at one end of it, said protuberance 11 defining a stop at the top and bottom ends of the fourth wall 7 when the panel reaches the open and closed limit positions of the fourth wall.

The flexible panel 8 is moved (in the direction indicated by the arrow F in Figures 1 and 2) between said open and closed limit positions of the fourth wall 7 by the above mentioned drive means 10.

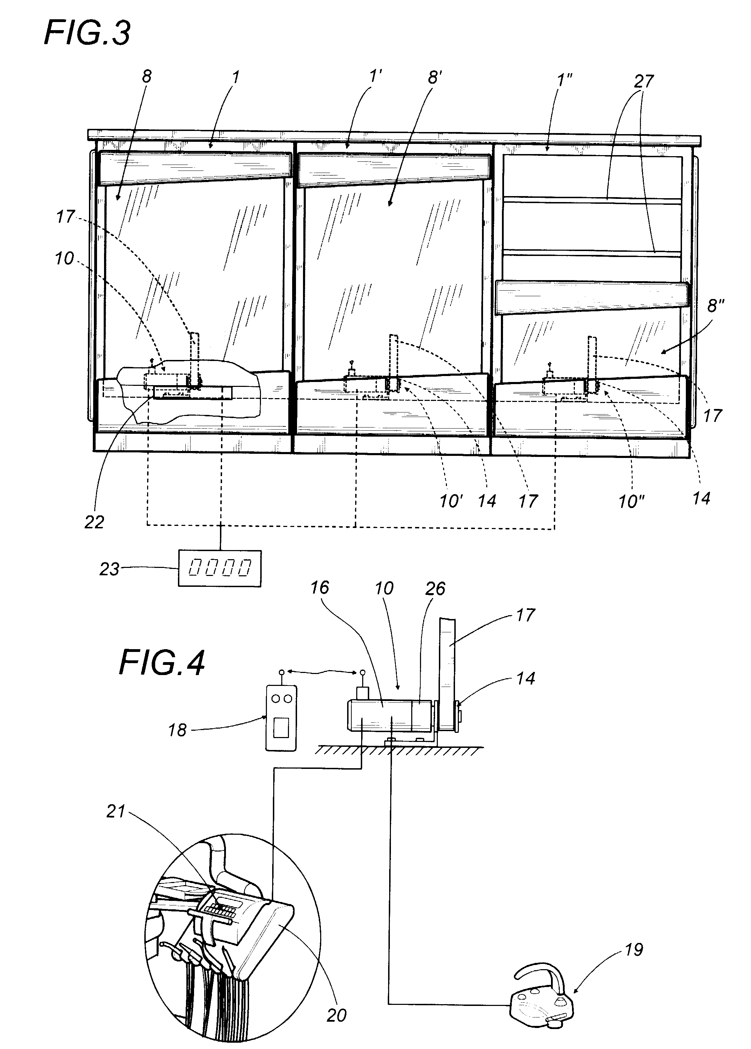

Looking in more detail, the drive means 10 consist of a pair of pulleys 14 and 15, of which one is equipped with a motor 16 (see also Figure 4). These pulleys 14 (with related motor 16) and 15 are connected to the body 1 at the top and bottom ends of said third wall 6 which forms the back wall of the body, opposite the fourth wall 7.

Closed in a loop around the pair of pulleys 14 and 15, there is a toothed belt 17, a branch 17r of which is joined by a fixing block 24 to one end of the flexible panel 8, so that the panel can, on command, move between the open and closed positions of the fourth wall 7. In practice, the pulleys 14 and 15, the belt 17 and the motor 16 are located in a gap 25 delimited by the third wall 6 and the vertical rear section of the pair of guides 9.

As can be seen in Figure 2, the drive means 10 can be operated in different ways. In one embodiment, the motor 16 may be operated by a remote control unit 18 consisting of a radio control. In another embodiment, the motor 16 may be connected to a pedal control unit forming part of the control system of a dental unit (not illustrated here since it is of a known type and does not strictly fall within the scope of the present invention).

In yet another embodiment, considering the fact that the dental surgery is likely to have a dental unit equipped with, amongst other things, a handpiece holder tablet 20 with a keyboard 21 for controlling various different functions, the motor 16 may be connected to the keyboard 21 so as to enable the dentist to open and close the furniture unit directly from the dental unit.

Yet another method of opening and closing the flexible panel 8 is by hand (preferably in the event of real necessity) using the above mentioned horizontal protuberance 11 as a handgrip. The manual sliding of the panel 8 is accomplished by applying a clutch unit 26 (see Figure 4) to the motor 16 so as to enable opening and closing in the event of a power failure in the surgery.

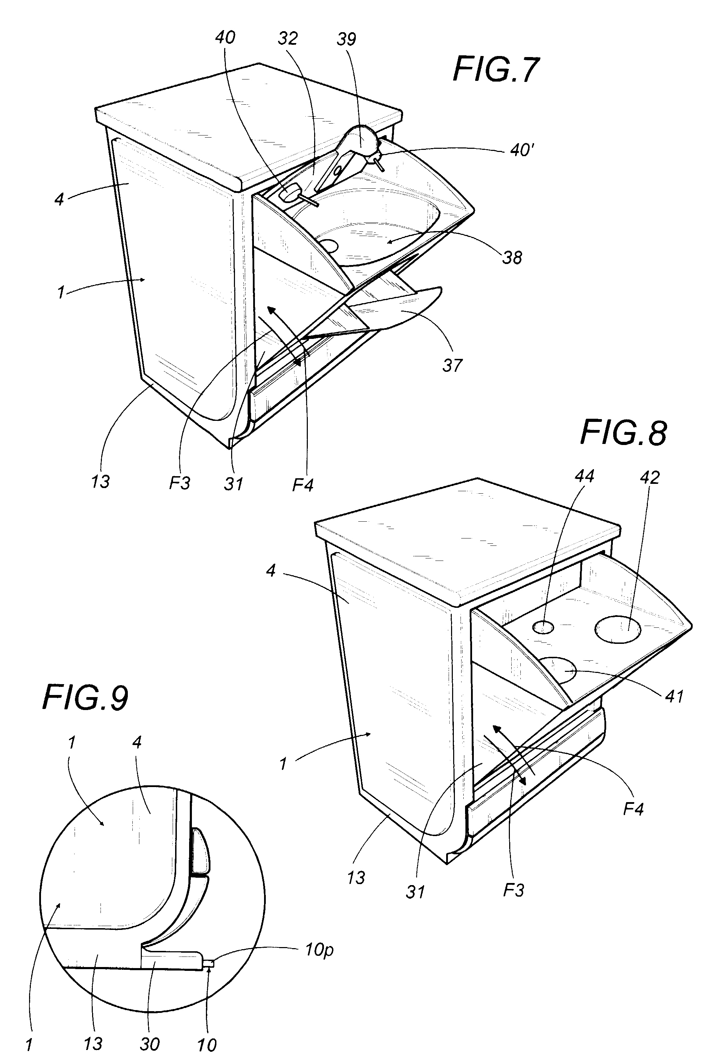

Another embodiment of the drive means 10 is illustrated in the detail shown in Figure 9. This embodiment envisages the use of a pedal switch 10p located on a front element 30, or skirting, covering the front of the base 13 of the furniture unit 1.

Figure 3 shows a composite item of furniture consisting, by way of example and without restricting the scope of the invention, of three furniture units such as those described above.

Obviously, there can be as many such furniture units as are necessary to meet the requirements of the surgery.

Each containing body 1, 1' and 1" has a flexible closing panel 8, 8' and 8" controlled by corresponding drive means 10, 10' and 10": in this item of furniture, one of said bodies, the one labelled 1, is equipped with a selector and control unit 22 connected to the drive means 10, 10' and 10" located on each body 1, 1' and 1" and designed to operate said drive means individually or simultaneously, depending on the requirements of the dentist.

Figure 3 also shows an additional element which the furniture unit disclosed herein can be equipped with: this element consists of a programmable timing unit 23 that can be applied to the drive means 10.

The unit 23 can be used to switch the drive means 10 after a preset delay time so as to automatically close the flexible panel 8. In other words, if the unit 23 is fitted, each time the furniture unit is opened to take out instruments and consumables needed for the current treatment session, the dentist need not remember to close the furniture unit since the unit 23 sends a control signal to the drive means 10 after a preset time in order to close the furniture unit.

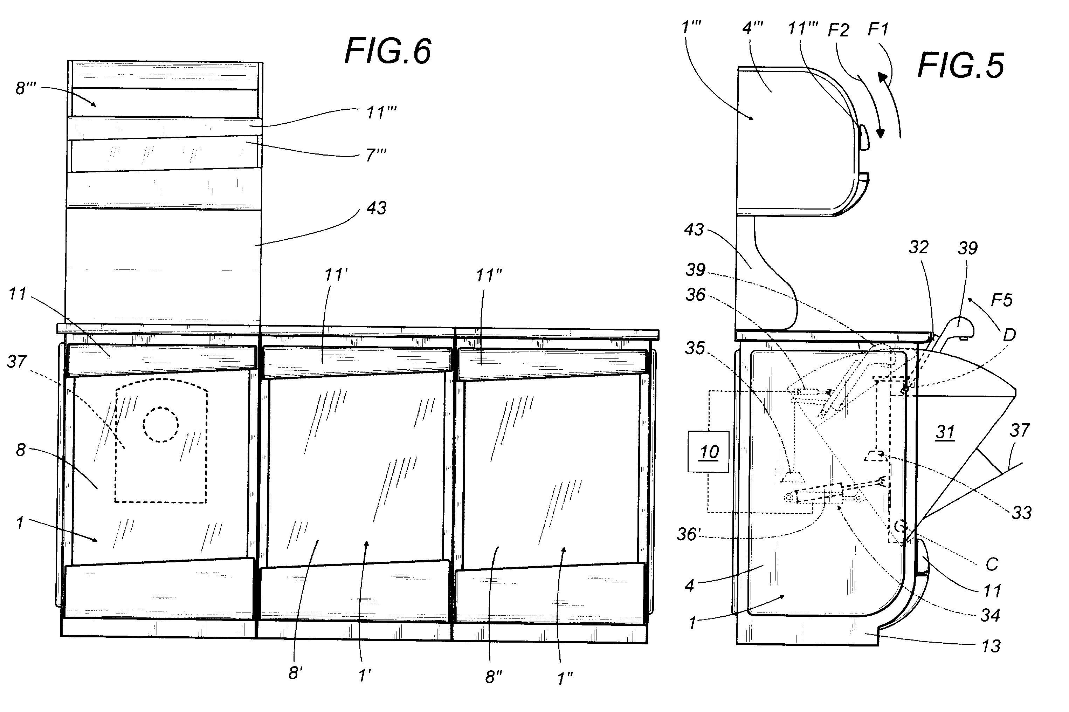

To confirm the efficacy of the modular structure described up to here, Figures 5 and 6 show another modular item of furniture consisting of three or more bodies 1, 1', 1", placed side by side in a parallelepipedal configuration, with another body 1"' forming a separate module that is rounded at the front, at the open zone 7.

Said body 1‴ may be connected to the top of one of the parallelepipedal bodies below it by a supporting column 43: the body 1"' is mounted at a convenient height to give the dentist easy access to a constant supply of disposable or frequently needed material such as, for example, masks, gloves, protective glasses.

The structure of the panel 8 and guides 9 is the same as that described above, basically differing only in the preferable opening and closing directions of the panel 8 (upwards to open, arrow F1, downwards to close, arrow F2).

In addition, the parallelepidal body or bodies 1, 1', 1", etc. may contain the above mentioned accessory elements (in particular, see Figures 7 and 8) which enable the provision of a complete furnishing scheme made according to high standards of hygiene and uniformity.

The compartment of each of the two furniture solutions illustrated in Figures 7 and 8 is equipped with a fold-away container 31, whose movements are synchronized with those of the panel 8 in such a way as to protrude at least partially from the open zone 7 when the panel 8 reaches the open position (this being the position illustrated in Figures 7 and 8) and to go back into the compartment when the panel 8 is returned to the closed position (see Figure 5).

The fold-away container 31 is connected to the body 1 at one end of it and at C (see dashed line in Figure 5), so as to enable it to tilt outwards to the position in which it protrudes from the open zone 7 (see arrow F3) and then go back to the position in which it is folded away inside the compartment (see arrow F4). In other terms, the fold-away container 31 has a pivot point C at the above mentioned first wall 2, forming the bottom of the body 1, so as to allow the top end of the container itself to protrude.

In the embodiment illustrated in Figure 7, the fold-away container 31 has an accessory block 32 pivoted to the container itself at one end, inside the compartment, at D: in this way, the accessory block 32 can be tilted away from the container 31 in a direction (see arrow F5) opposite that in which the container itself is simultaneously tilted outwards to protrude from the open zone 7.

The dashed line in Figure 5 shows schematically how both the accessory block 32 and the fold-away container 31 are equipped with first and second tilting means 33 and 34 that enable the block 32 to open and the container 31 to protrude from and reenter the compartment inside the body 1, according to a predetermined, synchronized sequence when the panel 8 is raised or lowered.

Both the first and the second tilting means 33 and 34 may be counterweights 35 connected, respectively, to the parts of the block 32 and of the container 31 that are inside the compartment.

Alternatively, the first and second means 33 and 34 for tilting the accessory block 32 and the container 31 may be pistons 36 and 36' connected, in the case of the block 32, to the container 31 and to the block itself at point D, while, in the case of the container 31, the piston 36' is connected to the container itself and to an inside wall of the body 1.

In Figure 7, the fold-away container 31 has on a horizontal surface of it a washbasin 38, while the accessory block 32 comprises a central tap 39 and two cleaning liquid dispensers 40, 40'. In addition to this, the front surface of the fold-away 37 container has a waste bin, which also tilts open when the container is completely outside the body 1.

In Figure 8, the fold-away container 31 comprises a pair of waste bins 41, 42, placed side by side on the horizontal surface, for the collection of different types of waste. An additional container 44 may also be fitted to collect used syringe needles and similar material.

A furniture unit as described herein achieves the above mentioned aims through a simple, economical structure consisting of a set of modular elements capable of being adapted to suit the requirements of the surgery and of the dentist.

The closing system with sliding flexible panel operated by remote control or by the control system of the dental unit not only eliminates the need for continual hand contact, thus reducing the risk of contamination by germs and bacteria, but also ensures a more effective, wear-resistant seal when closed.

The possibility of closing the flexible panel automatically means that the furniture unit remains open only for the time taken by the dentist to pick up or replace the instruments or consumables needed during the treatment session.

Moreover, the provision of fold-away elements further increases the range of uses that the furniture unit can be put to, allowing considerable saving of space in the surgery and increasing the safety and hygiene of the instruments used and of the surgery as a whole.

The invention described can be subject to modifications and variations without thereby departing from the scope of the inventive concept.

Moreover, all the details of the invention may be substituted by technically equivalent elements.

## Claims

1. A surgery furniture unit, especially for a dental surgery characterized in that it comprises:
- a body (1) forming a closed compartment designed to provide storage space and/or to mount accessory elements useful in the surgery, said body (1) having at least one open zone (7) giving access to said compartment;
- an element (8) used for closing the access zone (7) and consisting of a flexible panel operated on by guide means (9) connected to the body (1) and mounted in the compartment in such a way as to allow the panel (8) to slide between at least two limit positions of which one is a closed position where the panel (8) extends all the way along, and covers, the zone (7) and the other is an open position where the panel (8) has slid inside the compartment;
- means 10 for driving the panel (8) acting on the panel itself and designed to allow the latter to reach at least the zone (7) open and closed positions.

2. The furniture unit according to claim 1, characterized in that the means (10) for driving the flexible panel (8) comprise a pair of guides (9) joined to and extending along two opposite walls (4, 5) of the body (1).

3. The furniture unit according to claims 1 and 2, characterized in that the flexible panel (8) is a one-piece element that runs along the pair of guides (9) on two sides; said one-piece panel (8) moving to the zone (7) open position along a defined path and in such a way as to partly line the inside of the compartment.

4. The furniture unit according to claim 3, characterized in that said one-piece flexible panel (8) is made of metal.

5. The furniture unit according to claim 1, characterized in that the flexible panel (8) has a horizontal protuberance (11) at one end of it, said protuberance (11) defining a stop at the top and bottom ends of the zone (7) when the panel reaches the open and closed limit positions of said zone.

6. The furniture unit according to claim 1, characterized in that the body (1) defines a single module and is parallelepipedal in shape.

7. The furniture unit according to claim 1, characterized in that the body (1) defines a single module and has a rounded front, where the open zone (7) is located.

8. The furniture unit according to claims 1 to 3, characterized in that the pair of guides (9) extends along a U-shaped path partly inside the body (1), the curved portion of said guides being located in a gap (12) between a first horizontal wall (2) and a base (13) that supports the body (1).

9. The furniture unit according to claims 1 to 3, characterized in that the drive means (10) consist of a pair of pulleys (14, 15), of which one is equipped with a motor (16) and which are connected to the body (1) at the top and bottom ends of said third wall (6) which forms the back wall of the body (1), opposite the open zone (7); said pulleys (14, 15) having, closed in a loop around them, a toothed belt (17), a branch (17r) of which is joined to one end of the flexible panel (8), so that the panel can, on command, slide between the open and closed positions of the open zone (7).

10. The furniture unit according to claim 9, characterized in that the motor (16) of the pulley (14) is equipped with a clutch unit (26) designed to enable the flexible panel (8) to be operated by hand.

11. The furniture unit according to claim 1, characterized in that the means (10) for driving the flexible panel (8) can be operated by a remote control unit (18) consisting of a radio control.

12. The furniture unit according to claim 1, where the surgery is equipped with a dental unit with a pedal control unit (19), characterized in that the means (10) for driving the flexible panel (8) are connected to said pedal control unit (19).

13. The furniture unit according to claim 1, where the surgery is equipped with a dental unit with a handpiece holder tablet (20) having a keyboard (21) for controlling various different functions, characterized in that the means (10) for driving the flexible panel (8) are connected to said keyboard (21).

14. The furniture unit according to claim 1, characterized in that said drive means (10) can be operated by a pedal switch (10p) located on a front element (30) covering the front of the base (13) of the body (1).

15. The furniture unit according to claim 1, comprising at least two container bodies (1, 1'), each having a flexible panel (8, 8") controlled by corresponding drive means (10, 10'), characterized in that one of the bodies (1, 1') comprises a unit (22) for selecting and controlling the drive means (10, 10') and designed to operate said drive means (10, 10') individually or simultaneously.

16. The furniture unit according to claim 1, characterized in that the drive means (10) are equipped with a programmable timing unit (23) designed to switch the drive means (10) so as to automatically close the flexible panel (8) after a preset delay time.

17. The furniture unit according to claim 5, characterized in that the horizontal protuberance (11) is used as a handgrip to allow the flexible panel (8) to be operated manually.

18. The furniture unit according to claim 1, characterized in that the compartment within the body (1) is equipped with a fold-away container (31) that can move in synchrony with the panel (8) in such a way as to protrude at least partially from the open zone (7) when the panel (8) reaches the open position and vice versa.

19. The furniture unit according to claim 18, characterized in that the fold-away container (31) moves in synchrony with the panel (8) in such a way as to go back inside the compartment when the panel (8) moves back from the open position to the closed position.

20. The furniture unit according to claim 18, characterized in that the fold-away container (31) is connected to the body (1) at one end of it and at (C), so as to enable it to tilt outwards to the position in which it protrudes from the open zone (7) and then back into the compartment.

21. The furniture unit according to claim 20, characterized in that the fold-away container (31) is pivoted, at (C), to the above mentioned first wall (2), forming the bottom of the body (1).

22. The furniture unit according to claim 20, characterized in that the fold-away container (31) is equipped with an accessory block (32) pivoted to the container itself at one end, at (D), inside the compartment, so that it can be tilted away from the container (31), by appropriate means (33), in a direction opposite that in which the container itself is simultaneously tilted outwards to protrude from the open zone (7).

23. The furniture unit according to claim 20, characterized in that the fold-away container (31) is equipped with second tilting means (34) acting on it in synchrony with the movement of the panel (8) to open and/or close the open zone (7).

24. The furniture unit according to claims 22 and 23, characterized in that the first and second means (33, 34) for tilting the accessory block (32) and the fold-away container (31) consist of corresponding counterweights (35, 35') connected to them and located inside the compartment.

25. The furniture unit according to claims 22 and 23, characterized in that the first and second means (33, 34) for tilting the accessory block (32) and the fold-away container (31) consist of pistons (36, 36') each acting on the corresponding part and located inside the compartment.

26. The furniture unit according to claim 20, characterized in that the front of the fold-away container (31) is equipped with a waste bin (37) that tilts to open.

27. The furniture unit according to claims 20 to 22 characterized in that the fold-away container (31) has, on a horizontal surface of it, a washbasin (38) and in that the accessory block 32 comprises a central tap 39 and at least one cleaning liquid dispenser (40).

28. The furniture unit according to claim 20, characterized in that the fold-away container (31) comprises at least one waste bin (41).

29. The furniture unit according to claim 20, characterized in that the fold-away container (31) comprises at least two waste bins (41, 42) placed side by side and used to collect different types of waste.

30. The furniture unit according to claim 1, characterized in that it comprises a plurality of said bodies (1, 1', 1") constituting single modules placed side by side in a parallelepipedal configuration to form a single item of furniture, with another body (1‴) forming a separate module whose front open zone (7) is rounded and which is connected to the top of one of the parallelepipedal bodies (1) below it by a column (43).
